# EUROPÄISCHE PATENTANMELDUNG

(11) **EP 3 254 648 A1**
(43) Veröffentlichungstag der Anmeldung: **13.12.2017**
(21) Anmeldenummer: 17179001.7
(22) Anmeldetag: 30.07.2010
(51) Int. Cl.: A61F 5/56, A61F 2/848, A61F 2/86, A61M 39/28, A61F 2/04, A61F 2/95

(54) **FIXIERVORRICHTUNG ZUR FIXIERUNG EINES APNOE-STENTS IM ATEMWEG**

(30) Priorität: 31.07.2009 DE 202009010388 U
(62) Teilanmeldung aus: 10767917.7
(71) Anmelder: Düring, Klaus, 50226 Frechen (DE)
(72) Erfinder: Düring, Klaus, 50226 Frechen (DE); Pfeffer, Joachim Georg, 52070 Aachen (DE)
(74) Vertreter: Schüssler, Andrea

(57) **Zusammenfassung**

Die Erfindung betrifft eine Fixiervorrichtung zur Fixierung eines Apnoe-Stents im Atemweg, wobei die Fixiervorrichtung (10) eine Fixiereinrichtung aufweist, die an einem proximalen Ende (2) des Apnoe-Stents (1) fixierbar ist. Die Fixiervorrichtung zeichnet sich dadurch aus, dass die Fixiereinrichtung zwei Klemmelemente (11, 12) aufweist, zwischen welchen das proximale Ende (2) des Apnoe-Stents (1) fixierbar ist.

Weiterhin betrifft die Erfindung ein Verbindungselement zum Verbinden eines Apnoe-Stents (1) und einer Einführstange (5), einen Reinigungsschlauch zur Aufnahme eine Apnoe-Stents und ein System zur Schienung des Atemweges, umfassend einen Apnoe-Stent und eine Fixiervorrichtung.

## Beschreibung

Die vorliegende Erfindung betrifft eine Fixiervorrichtung zur Fixierung eines Apnoe-Stents im Atemweg.

Krankheitsbilder, die auf dem zumindest partiellen Verschluss von Hohlräumen, Organwegen und/oder Gefäßen beruhen, nehmen zivilisationsbedingt stark zu. Ein wichtiger Bereich solcher Krankheitsbilder bezieht sich auf die Atemwege. So ist die obstruktive Schlafapnoe eine lebensbedrohliche Erkrankung, die aus dem Verschluss der hinteren Atemwege durch Muskelerschlaffung während des Schlafes resultiert. Durch wiederholten, teilweise sehr häufigen kurzzeitigen Atemstillstand wird der Patient während des Schlafs nicht ausreichend mit Sauerstoff versorgt. Im Extremfall kann dies tödlich sein. Zivilisationsbedingte Faktoren, wie z.B. starke Fettleibigkeit oder übermäßiger Alkoholgenuss, verstärken das Erkrankungsrisiko deutlich. Das Schnarchen ist eine leichtere Effektausprägung als die Schlafapnoe, resultierend aus den gleichen organischen Ursachen. Während das Schnarchen den meisten Menschen bekannt ist, werden die schwerwiegenden gesundheitsgefährdenden Folgen des starken Schnarchens und der Schlafapnoe oft nicht registriert, da der Patient sich der Folgen der organischen Mangelerscheinungen nicht bewusst ist.

Aus der WO 2007/065408 A2 geht eine Vorrichtung zur Schienung und/oder Offenhaltung eines Hohlraumes, eines Organweges und/oder eines Gefäßes im menschlichen oder tierischen Körper mit zumindest einem komprimierbaren und selbstexpandierenden Stent, der zumindest einen aufgeweiteten Bereich aufweist, hervor. Dieser Stent wird mittels einer nicht näher beschriebenen Befestigungsplatte gegen ein zu tiefes Eindringen in den Atemweg fixiert. Auf die WO 2007/065408 A2 wird hiermit vollinhaltlich Bezug genommen. Ein Stent, wie er in der WO 2007/065408 A2 beschrieben ist, wird im folgenden als Apnoe-Stent bezeichnet.

Dieser Apnoe-Stent hat sich bei ersten klinischen Tests zur Behandlung von obstruktiver Schlafapnoe sehr bewährt.

In der US 2006/00 70 626 A1 ist ein Verfahren und eine Vorrichtung zum Behandeln von Schlafapnoe beschrieben. Die Vorrichtung umfasst einen Endotrachealstent der endoskopisch in die Trachea einbringbar ist. Das proximale und das distale Ende dieses Stents kann z. B. mit Haken, Stacheln oder Klips versehen sein, um ihn in der Trachea anzuordnen.

Aus der DE 10 2004 012 351 A1 geht eine Vorrichtung zur Rekanalisierung eines Hohlraums, Organwegs oder Gefäßes hervor. Die Vorrichtung zur Rekanalisierung ist ein expandierbarer Stent.

In der DE 102 40 725 A1 ist eine Vorrichtung zur Verhinderung des Schnarchens und der Atemstillstände während des Schlafs beschrieben. Diese Vorrichtung beschreibt ebenfalls eine Art Stent, wobei das Ende welches aus der Nase herausragt und welches der Arretierung dient, bogenförmig geformt und mit einer Eingangsöffnung versehen ist, sodass die Luftzufuhr durch die Nase nicht beeinträchtigt wird.

In der US 2009/00 44 814 A1 ist ein Widerhakensystem beschrieben, das zum Arretieren im Gewebe mit Haken versehen ist und chirurgisch implantiert wird.

Aus der DE 202 16 956 U1 geht eine Vorrichtung zur Verbesserung der Nasenatmung und Verhinderung des Schnarchens hervor. Diese Vorrichtung umfasst zwei kurze rohrförmige Elemente, die über ein Verbindungselement miteinander gekoppelt sind und in die Nasenflügel einsetzbar sind, wodurch das Schnarchen verhindert und die Nasenatmung verbessert werden soll. Diese Vorrichtung wird nur am Naseneingang eingesetzt und erstreckt sich nicht bis in den Rachenraum.

In der WO 98/23233 A1) ist ein Schlauch zum Verhindern von Schlafapnoe beschrieben.

Aus der WO 2008/0 58 367 A1 geht eine erweiterbare Vorrichtung zum Einbringen in die Nase hervor. Diese Vorrichtung umfasst zwei spiralförmige gewickelte rohrförmige Elemente, die in die Nasenflügel einbringbar sind und die mittels eines Verbindungselements miteinander gekoppelt sind.

In der US 61 06 548 A ist ein lediglich einmal komprimierbarer und expandierbarer Stent beschrieben.

Aus der US 2001/00 44 647 A1 geht ein expandierbarer Endoluminalstent hervor.

In der DE 102 33 862 A1 ist ein System zum Verbinden einer rohrförmigen Leitung insbesondere eines Kunststoffschlauchs mit einer Verbindungsvorrichtung beschrieben. Die Verbindungsvorrichtung weist sich in Längsrichtung erstreckende Klipse auf, die in entsprechende Nasen der rohrförmigen Leitung eingreifen.

Aus der EP 05 17 000 A2 geht eine Schlauchklemme für medizinische Zwecke zum Abklemmen eines Schlauches hervor.

In der DE 10 2004 023 559 A1 ist ein Herzkatheter mit einem entsprechenden Stent beschrieben.

Aus der EP 1 181 906 B1 geht ein Katheter zum Einführen eines Stents hervor.

In der D15 (DE 100 12 852 A1) ist ebenfalls ein Katheter zum Einführen expandierbaren Stents beschrieben.

Aufgabe der vorliegenden Erfindung ist es, eine kostengünstige Vorrichtung zu schaffen, die sicher und zuverlässig ein Eindringen des Apnoe-Stents in den Atemweg nach dem Einführen bzw. während des Schlafens verhindert und einfach handhabbar ist.

Die Aufgabe wird mit einer Vorrichtung mit den Merkmalen des Anspruchs 1 gelöst. Vorteilhafte Ausgestaltungen der Erfindung sind in den Unteransprüchen angegeben.

Erfindungsgemäß ist eine Fixiervorrichtung zum Fixieren eines Apnoe-Stents im Atemweg vorgesehen. Die Fixiervorrichtung weist eine Fixiereinrichtung auf, die an einem proximalen Ende des Stents fixierbar ist und dadurch die Position des Stents im Atemweg fixiert. Die Fixiervorrichtung zeichnet sich dadurch aus, dass die Fixiereinrichtung zwei Klemmelemente aufweist, zwischen welchen das proximale Ende des Apnoe-Stents fixierbar ist.

Diese Fixiereinrichtung kann einfach mit einer Hand gehandhabt werden, so dass mit der anderen Hand beim Fixieren der Fixiereinrichtung am Apnoe-Stent das proximalen Ende des Apnoe-Stents gehalten werden kann. Die einfache Handhabung ist sehr wichtig, da die Position des proximalen Endes des Apnoe-Stents am Rand oder außerhalb des Sichtfeldes der jeweiligen Person liegt, die den Apnoe-Stent benutzt.

Ein zu tiefes Eindringen in den Atemweg eines Patienten wird sicher und zuverlässig verhindert, da die Fixiervorrichtung an den Nasenflügeln des Patienten ansteht und der Stent somit nicht tiefer in den Atemweg eines Patienten hineinrutschen kann.

Gleichermaßen kann durch eine Befestigung der Fixiervorrichtung am Kopf sicher und zuverlässig das Herausrutschen des Stents aus dem Atemweg verhindert werden.

In einer bevorzugten Ausführungsform der vorliegenden Erfindung weist die Fixiervorrichtung eine erste und eine zweite Klemmbacke auf, die über ein Gelenk miteinander verbunden sind. Die beiden Klemmbacken sind derart aneinanderliegend anordbar, dass ein proximales Ende des Apnoe-Stents zwischen den beiden Backen fixierbar ist. Die beiden Klemmbacken sind vorzugsweise mit einer Rast- oder Schnappeinrichtung aneinander fixierbar.

Durch die beiden Klemmbacken wird eine einfache Fixierung des Apnoe-Stents im Atemweg gewährleistet. Des weiteren ermöglichen die Klemmbacken ein schnelles und einfaches Befestigen und Lösen der Fixiervorrichtung am proximalen Ende des Stents.

Aufgrund des einfachen Aufbaus ist die Fixiervorrichtung einfach und kostengünstig herzustellen.

Die Erfindung wird beispielhaft anhand der Zeichnungen näher erläutert. Es zeigen dabei schematisch:
- Figur 1a, b, c: einen Apnoe-Stent zusammen mit einer Einführstange und einem Einführschlauch in perspektivischer Ansicht ,
- Figur 2: eine erfindungsgemäße Fixiervorrichtung in einer Seitenansicht im offenen Zustand,
- Figur 3: die Fixiervorrichtung aus Figur 2 in einer seitlichen Ansicht im geschlossenen Zustand,
- Figur 4: die Fixiervorrichtung aus Figur 2 in einer Draufsicht im offenen Zustand,
- Figur 5a, b, c: eine erste Klemmbacke der Fixiervorrichtung in verschiedenen Ansichten,
- Figur 6a, b, c: eine zweite Klemmbacke der Fixiervorrichtung in verschiedenen Ansichten,
- Figur 7: den Endbereich der Einführstange aus Figur 1, an dem eine Buchse angeordnet ist in einer Seitenansicht,
- Figur 8: das proximale Ende des Apnoe-Stent aus Figur 1 mit einem kugelförmigen Verbindungselement in einer Seitenansicht,
- Figur 9: einen Endbereich des Einführschlauchs aus Figur 1 in einer Schnittdarstellung,
- Figur 10a: das kugelförmige Verbindungselement in einer Seitenansicht
- Figur 10b: das kugelförmige Verbindungselement aus Figur 10a in einer seitlich geschnitten Ansicht entlang der Linie A-A
- Figur 11a: die Buchse aus Figur 7 in einer weiteren Seitenansicht, und
- Figur 11b: die Buchse aus Figur 11a in einer weiteren seitlich geschnitten Ansicht entlang der Linie A-A, und

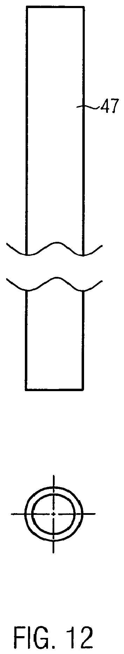

- Figur 12: einen erfindungsgemäßen Reinigungsschlauch.

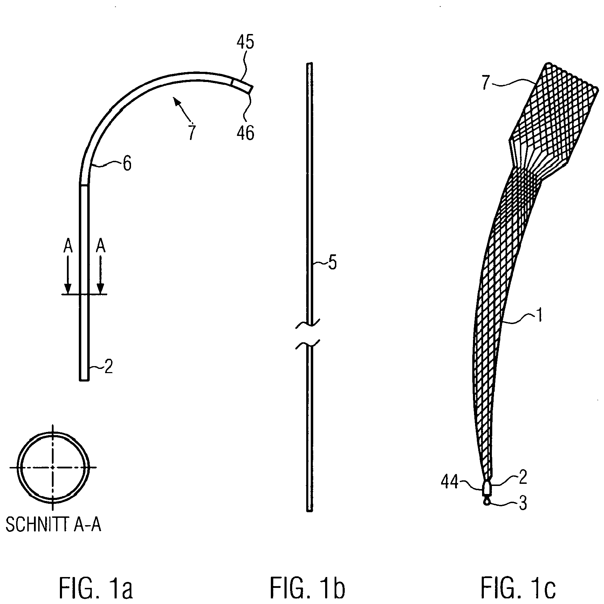

Ein aus der WO 2007/065408 A2 bekannter Apnoe-Stent 1 (Fig. 1 c), auf die hiermit vollinhaltlich Bezug genommen wird, ist dreiphasig ausgebildet.

Eine distale Phase des Stents bildet einen aktiven Teil des Apnoe-Stents 1 aus. Diese distale Phase ist rohrförmig ausgebildet und derart aufweitbar, dass der Atemweg offen gehalten wird.

Eine proximale Phase des Stents ist zur Fixierung des Stents im Nasenbereich vorgesehen. Die proximale Phase ist trichterförmig ausgebildet wobei sie sich von einem proximalen zu einem distalen Ende hin aufweitet.

Die distale und die proximale Phase sind als Geflecht ausgebildet, bei dem sich Drähte bzw. Fasern bzw. Fäden kreuzen. Im Folgenden wird lediglich der Begriff Draht verwendet, wobei hiermit auch Fasern oder Fäden gemeint sind.

Jeder einzelne in Richtung zum distalen Ende des Stents verlaufende Draht wird am distalen Ende der distalen Phase zurück zum proximalen Ende des Stents geführt. Die hierdurch erzeugten Biegungen werden als runde Enden bezeichnet. Diese weisen im geöffneten Zustand des Stents einen Durchmeser im Bereich von ca. 1 - 2 mm auf. Auf diese Weise wird ein distales Ende bereitgestellt, an dem keine einzelnen Drahtenden frei liegen oder mit einem weiteren Element miteinander verbunden werden müssen. Die Verbindung des Drahtes bzw. der Drähte des Stents kann durch Verkrimpen am proximalen Ende des Stents erfolgen. Durch die runden Enden werden Verletzungen der Atemwege durch das distale Ende des Apnoe-Stents vermieden.

Eine Übergangsphase des Stents ist zum Verbinden der proximalen Phase mit der distalen Phase vorgesehen. Die Übergangsphase ist durch Verzwirbelung der Drähte des Stents zu verzwirbelten Strängen ausgebildet. Ein jeder Strang umfasst mindestens zwei Drähte. Die einzelnen Stränge verlaufen etwa in Axialrichtung des Apnoe-Stents, ohne sich zu kreuzen. Hierdurch weist der Übergangsbereich in Radialrichtung eine hohe Flexibilität auf, so dass innerhalb einer kurzen Längserstreckung eine starke radiale Aufweitung von der proximalen zur distalen Phase bewirkt wird. Dadurch wird sichergestellt, dass über die gesamte Länge der distalen Phase in etwa ein konstanter Durchmesser ausgebildet wird.

Am proximalen Ende 2 des Apnoe-Stents ist ein Verbindungs- oder Kopplungselement 3 angeordnet, das mit einem Verbindungs- oder Kopplungselement 4 einer Einführstange 5 koppelbar ist. Das Verbindungselement 4 ist an einem Ende der Einführstange 5 angeordnet. Im vorliegenden Ausführungsbeispiel ist das Verbindungselement 4 der Einführstange 5 als Buchse und das Verbindungselement 3 des Apnoe-Stents 1 als formschlüssig in die Buchse passende Kugel ausgebildet.

Zum Einführen des Apnoe-Stents 1 in den Atemweg wird der Apnoe-Stent 1 mit Hilfe der Einführstange 5 in einen Einführschlauch 6 eingeführt und komprimiert. Hierzu wird die Einführstange 5 durch den Einführschlauch 6 geschoben, so dass der Apnoe-Stent 1 von der Einführstange 5 in den Einführschlauch 6 gezogen wird.

Für die Anwendung dieser Vorrichtung zur Behandlung von Schnarchen und/oder Schlafapnoe wird der Stent 1 in im Einführschlauch 6 komprimierten Zustand in eines der beiden Nasenlöcher eingeführt und vorzugsweise bis maximal zum Kehlkopfdeckel vorgeschoben und an seinem Anwendungsort platziert. Durch Zurückziehen des Einführschlauches 6 wird der Stent 1 frei gesetzt und expandiert selbstständig in seine vorbestimmte Größe. Dadurch wird das Zusammenfallen des Schlundes und das Verschließen der Atemwege verhindert. Ein freier Luftstrom und eine normale Atmung wird dadurch ermöglicht. Nach der korrekten Anordnung des Apnoe-Stents 1 im Atemweg wird die Verbindung mit der Einführstange gelöst.

Dieser Stent 1 wird mittels einer erfindungsgemäßen Fixiervorrichtung 10 gegen ein zu tiefes Eindringen in und ein Herausrutschen aus dem Atemweg gesichert bzw. fixiert. Mit dieser kann die korrekte Einführungstiefe des Apnoe-Stents festgelegt werden und der Apnoe-Stent 1 gegen unbeabsichtigtes Hineinrutschen in den Atemweg gesichert werden.

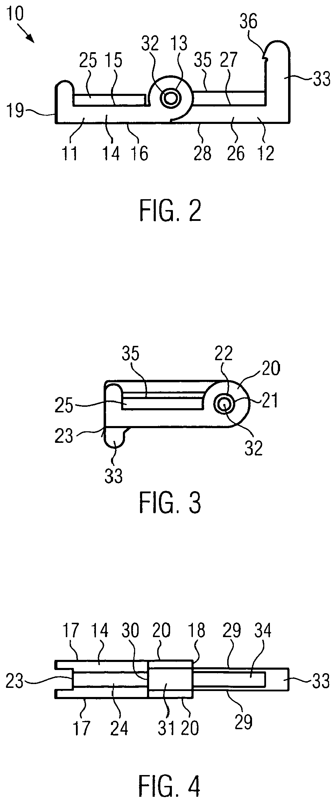

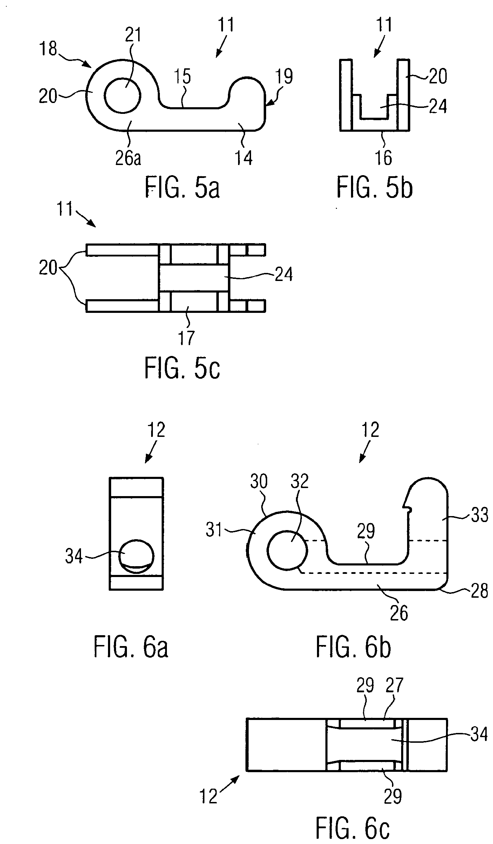

Die erfindungsgemäße Fixiervorrichtung 10 umfasst eine erste und eine zweite Klemmbacke 11, 12, die über ein Gelenk 13 miteinander verbunden sind. Die erste und die zweite Klemmbacke 11, 12 bilden eine Fixiereinrichtung aus (Fig. 3, 4, 5a, b, c, 6a, b, c).

Die erste Klemmbacke 11 wird im folgenden als Basisbacke bezeichnet. Die Basisbacke 11 weist eine rechteckförmige Basiswandung 14 auf. Die Basiswandung 14 umfasst eine Oberseite 15, eine Unterseite 16 und von der Basiswandung 14 erstrecken sich an deren Längsseiten zwei Seitenwandungen 17 nach oben, so dass die Basiswandung 14 und die zwei Seitenwandungen 17 eine U-förmige Ausnehmung 24 begrenzen. Die beiden gegenüberliegenden Enden der quaderförmigen Basisplatte 14 werden als Gelenkseite 18 und Verschlussseite 19 bezeichnet.

An der Gelenkseite 18 der quaderförmigen Basisplatte 14 sind zwei Ringscheibenelemente 20 beabstandet voneinander angeformt, die bündig zu den Außenflächen der Seitenwandungen 17 ausgebildet sind. Die Ringscheibenelemente 20 weisen jeweils eine kreisförmige Durchgangsöffnung 21 auf, die zueinander fluchten. Die Durchgangsöffnungen 21 sind jeweils zur Aufnahme einer Rohrwelle 22 ausgebildet. Die Rohrwelle ist drehbar in den Durchgangsöffnungen 21 angeordnet und bündig zu den Außenflächen der Seitenwandungen ausgebildet. Über die Rohrwelle 22 ist die erste Klemmbacke 11 gelenkig mit der zweiten Klemmbacke 12 verbunden.

An der Verschlussseite 19 erstrecken sich die Seitenwandungen 17 über den stirnseitigen Rand der Basiswandung 14 hinaus, so dass sie in der Draufsicht auf die Basisbacke 11 (Fig. 4) die U-förmige Ausnehmung begrenzen. Die Seitenwandungen sind in diesem Bereich ein Stück nach oben gezogen.

Die stirnseitige Kante der Basiswandung 14 an der Unterseite 16 wird im folgenden als Verschlusskante 23 bezeichnet.

In der Nut 24 ist ein quaderförmiger Fixierblock 25 angeordnet. Der quaderförmige Fixierblock 25 ragt in etwa 3 mm auf der Oberseite 15 der Basiswandung 14 heraus. Der quaderförmige Fixierblock 15 ist aus einem Kunststoff, insbesondere Silikon, ausgebildet uund bildet ein Fixierelement aus.

Die zweite Klemmbacke 12 ist L-förmig ausgebildet und weist einen quaderförmigen Querschnitt auf. Die zweite Klemmbacke 12 umfasst einen langen Schenkel 26 und einen daran im rechten Winkel angeformten kurzen Schenkel, der im folgenden als Fixierschenkel 33 bezeichnet wird. Der lange Schenkel 26 weist eine Basiswandung 26a mit einer Oberseite 27 und einer Unterseite 28 und zwei Seitenwandungen 29 auf, die sich von der Oberseite 27 nach oben erstrecken und eine Nut zwischen sich begrenzen.

Das dem Fixierschenkel 33 gegenüberliegende Ende des langen Schenkels 26 wird als Gelenkseite 30 bezeichnet. An dieser Gelenkseite 30 ist ein rohrförmiger Gelenkkörper 31 angeformt. Der Gelenkkörper 31 bildet ein Rohr mit zwei Seitenwandungen 29 und einer kreisförmigen Durchgangsöffnung 32, wobei die Länge des Rohrs der lichten Weite zwischen den beiden Ringscheibenelementen 20 entspricht. In der Durchgangsöffnung des Gelenkkörpers ist die Rohrwelle 22 angeordnet. Die Rohrwelle 22 lagert drehbar in den Durchgangsöffnungen 21 der Ringscheibenelemente 20 und in der Durchgangsöffnung 32 des Gelenkkörpers 31.

Die Rohrwelle kann auch lediglich drehbar in den Durchgangsöffnungen 21 der Ringscheibenelemente 20 oder in der Durchgangsöffnung 32 des Gelenkkörpers 31 angeordnet sein. In jedem Fall wird die Rohrwelle derart fixiert, dass ein selbstständiges Herausrutschen aus der Fixiervorrichtung vermieden wird.

Im Fixierschenkel 33 ist ein Durchgangsloch 34 ausgebildet, das mit der Nut des langen Schenkels 26 fluchtet. Im rohrförmigen Gelenkkörper 31 ist eine Ausnehmung eingebracht, die auch mit der Nut des langen Schenkels 26 fluchtet. Im Durchgangsloch 34, der Nut des langen Schenkels 26 und der Ausnehmung des Gelenkkörpers 31 ist ein Fixierschlauch 35 aufgenommen. Der Fixierschlauch 35 bzw. das Fixierelement ist aus Kunststoff, insbesondere Silikon, ausgebildet.

Am freien Ende des Fixierschenkeis 33 ist eine in Richtung zum Gelenkkörper 31 zeigende Rastnase 36 ausgebildet. Die Rastnase 36 ist derart ausgebildet, dass sie die Fixierkante 23 hintergreift, wenn die beiden Schenkel zusammengedrückt werden. Die Rastnase 36 rastet dann hinter der Fixierkante 23 ein und hält die Fixiervorrichtung 10 in einem geschlossenen Zustand. Die Rastnase 36 und die Fixierkante bilden so ein Verschlusselement aus. Im geschlossenen Zustand der Fixiervorrichtung sind die Basisbacke 11 und die zweite Klemmbacke 12 etwa parallel zueinander angeordnet, wobei der Fixierblock 25 und der Fixierschlauch 35 aufeinander gepresst werden.

Zwischen dem Fixierblock 25 der Klemmbacke 11 und dem Fixierschlauch 35 der Klemmbacke 12 ist das proximale Ende 2 des Apnoe-Stents 1 anordbar. Durch Aufeinanderpressen und Deformierung des rohrförmigen Fixierschlauchs 35 und des quaderförmigen Fixierblocks 25 wird das proximale Ende 2 des Stents 1 in der Fixiervorrichtung 10 ortsfest angeordnet. Das aus einem Nitinolgeflecht ausgebildete proximale Ende des Apnoe-Stents wird zwischen dem Fixierschlauch 35 und dem Fixierblock 25 bzw. den Fixierelementen flachgedrückt bzw. deformiert und fixiert, ohne dabei beschädigt oder dauerhaft verformt zu werden. Da die Basisbacke 11 und die Klemmbacke 12 im geschlossenen Zustand miteinander verspannt sind, wird ein Verrutschen des proximalen Endes 2 des Apnoe-Stents verhindert.

Der Fixierblock 25 und der Fixierschlauch 35 bilden geradlinige Klemmkanten bzw. Klemmflächen ohne Verzahnung oder dergleichen. Hierdurch kann ein Apnoe-Stent mehrfach zwischen der Basisbacke 11 und der Klemmbacke 1 eingeklemmt werden, ohne dabei beschädigt zu werden.

Das durch die Rastnase 36 und die Fixierkante 23 ausgebildete Verschlusselement liegt außerhalb des Kontaktbereichs zwischen den beiden Klemmelementen 11, 12. Im vorliegenden Ausführungsbeispiel ist das Verschlusselement an der Unterseite 16 der Basiswandung 14 der Basisbacke 11 und somit auf der vom Kontaktbereich abgewandten Seite der Basisbacke 11 angeordnet. Durch den Abstand zwischen dem Verschlusselement und dem Kontaktbereich ist die Gefahr gering, dass ein Bereich des Apnoe-Stents im Verschlusselement zwischen der Rastnase 36 und der Fixierkante 23 eingeklemmt und beschädigt wird.

Durch die Dimensionierung der Höhe des Fixierblocks 25 und des Durchmessers des Fixierschlauchs 35 wird ein Anpressdruck definiert, mit der das proximale Ende des Apnoe-Stents fixiert wird. Beim Fixieren wird der Fixierschlauch 35 vom Fixierblock zusammengedrückt. Je geringer die Höhe des Fixierblocks 25 und je geringer der Durchmesser des Fixierschlauchs 35, desto geringer ist der Anpressdruck. Je größer die Höhe des Fixierblocks 25 und je größer der Durchmesser des Fixierschlauchs 35, desto größer ist der Anpressdruck. Zusätzlich kann der Anpressdruck durch die Härte der Materialien für den Fixierblock 25 und den Fixierschlauch 35 eingestellt werden. Aus dem Anpressdruck resultiert eine Schließkraft, die überwunden werden muss, um die Basisbacke und die Klemmbacke in ihren geschlossenen Zustand zusammen zu drücken.

Entscheidend bei der Dimensionierung ist, dass der Benutzer die beiden Klemmbacken noch einfach, d.h. mit akzeptablem Kraftaufwand zusammendrücken kann. Hierfür muss der Benutzer die Schließkraft und eine Arretierkraft überwinden. Die Arretierkraft ist die Kraft, die notwendig ist, um den Fixierschenkel soweit zurückzubiegen, dass die Rastnase die Fixierkante nicht mehr hintergreift. Sie ist somit von der Elastizität und der Festigkeit des Materials, aus dem der Fixierschenkel ausgebildet ist abhängig. Zum Öffnen der Fixiervorrichtung muss lediglich die Arretierkraft überwunden werden.

Somit sind die Basisbacke 11, die Klemmbacke 12, der Fixierblock 25 und der Fixierschlauch 35 bzgl. Größe, Geometrie und Material derart zu auszulegen, dass das proximale Ende des Apnoe-Stents ausreichend fixiert wird, ohne dabei beschädigt zu werden, und außerdem ein Benutzer die Fixiervorrichtung einfach und leicht, vorzugsweise einhändig, verschließen kann. Über die Dimensionierung der Basisbacke 11, der Klemmbacke 12, des Fixierblocks 25 und des Fixierschlauchs 35 ist der Anpressdruck und die Fixierkraft variabel einstellbar.

Die Geometrie und die Größe der beiden Klemmbacken und die Geometrie der Rastnase wirken sich ebenfalls auf die Haltekraft aus. Die Dimensionierung der Fixiervorrichtung ergibt sich aus dem optimalen Kompromiss zwischen einer ausreichenden Größe für eine benutzerfreundlich Handhabung der Fixiervorrichtung und möglichst kompakten Abmessungen, um den Benutzer beim Schlafen möglichst wenig zu beeinträchtigen.

Insbesondere sind die Klemmflächen der Klemmbacken derart ausgebildet, dass das Nitinolgeflecht beim Schließen der Fixiervorrichtung nicht beschädigt wird. Die Länge der Klemmflächen muss mindestens der Breite des zusammengedrückten Nitinolgeflechts entsprechen, um ein Übereinanderknicken des Nitinolgeflechts und somit Beschädigungen zu vermeiden. Eine oder beide Oberflächen der Klemmbacken sind als elastische Flächen ausgebildet. Die Oberflächen sind eben bzw. plan oder konvex ausgebildet, d.h. sie weisen keine scharfen Kanten auf, um das Nitinolgeflecht nicht zu beschädigen. Es kann auch vorgesehen sein, dass die Oberfläche einer der beiden Klemmbacken eine raue Oberfläche mit einer Strukturierung von bis zu 1/10 mm aufweist, um das Nitionolgeflecht gegen Verrutschen zu sichern. Es können anstatt des Fixierblocks und des Fixierschlauchs aus Silikon auch geschlossenporige Schaumstoffe vorgesehen sein, die einstückig mit den Klemmbacken verbunden sind. Der auf das Nitinolgeflecht aufgebrachte Anpressdruck kann entlang einer Linie oder über einen Teil oder die gesamte Fläche der Klemmbacken aufgebracht werden. Der Apnoe-Stent ist quer zu den Klemmbacken in der Fixiervorrichtung angeordnet. Die Klemmung des Nitinolgeflechts erfolgt somit quer zur Längsrichtung des Apnoe-Stents.

Beim Gebrauch steht die Fixiervorrichtung 10 an der Nase eines Patienten im Bereich um ein Nasenloch herum an, wodurch ein zu tiefes Hineinrutschen des Apnoe-Stents in den Patienten sicher und zuverlässig vermieden wird.

In der Durchgangsöffnung bzw. Aufnahme 32 ist eine Befestigungseinrichtung 37 zur Anordnung der Fixiervorrichtung am Kopf des Patienten anordbar, um die Fixiervorrichtung zu befestigen und ein Herausrutschen des Apnoe-Stents aus dem Atemweg zu verhindern. Die Befestigungseinrichtung 37 kann beispielsweise als weiches und/oder elastisches Kopfband ausgebildet sein, welches sich durch die zylindrische Durchgangsöffnung bzw. Aufnahme 32 hindurch erstreckt und um den Kopf herum angeordnet wird. Es ist auch eine Befestigungseinrichtung möglich, die in der Art von Brillenbügeln bzw. Schlaufen um die Ohren eines Patienten anordbar ist. Dadurch, dass als Aufnahme eine Durchgangsöffnung vorgesehen ist, kann die Verbindung mit der Befestigungseinrichtung 37 nicht versehentlich geöffnet bzw. gelöst werden. Eine weitere Befestigungseinrichtung kann in der Form eines Stiftes, der formschlüssig in die Durchgangsöffnung 32 eingesteckt werden kann und einen aus der Durchgangsöffnung vorstehenden Vorsprung aufweist, der mittels eines Pflasters am Benutzer fixiert wird, ausgebildet werden. Dieser Vorsprung ist vorzugsweise plattenförmig ausgebildet.

Des Weiteren ist ein Verbindungselement 39 vorgesehen, das eine lösbare Verbindung zwischen der Einführstange 5 und dem proximalen Ende 2 des Apnoe-Stents 1 gewährleistet. Das Verbindungselement 39 umfasst das Verbindungselement 3 am proximalen Ende 2 des Apnoe-Stents 1 und das Verbindungselement 4 am distalen Ende der Einführstange 5 (Fig. 1a, 7, 8, 10a, 10b, 11a, 11b).

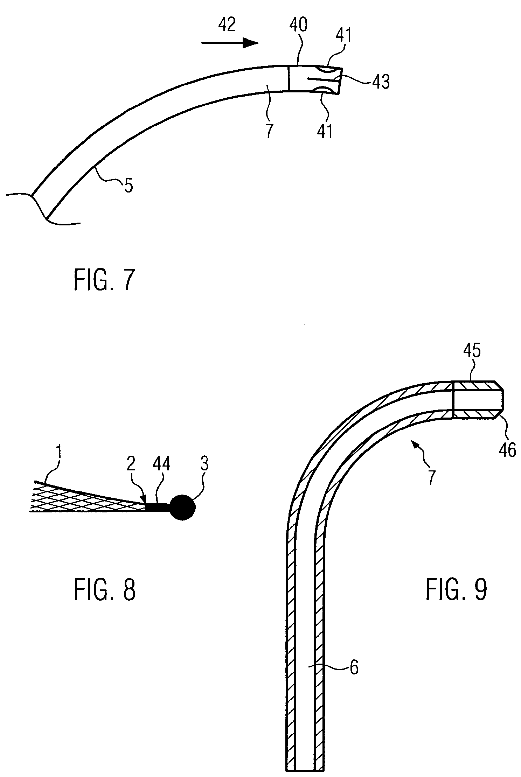

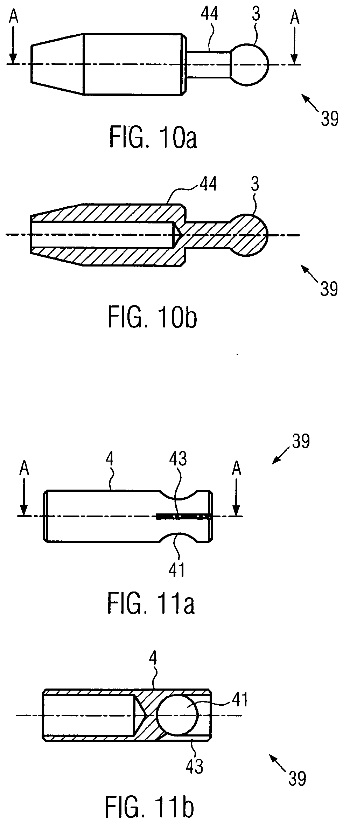

Die Einführstange 5 ist aus einem Kunststoff ausgebildet (Fig. 1a, 7). Am distalen Ende 7 der Einführstange 5 ist vorzugsweise einstückig eine rohrförmige Buchse 4 angeformt (Fig. 7, 11a, 11b). Die Buchse 4 weist zwei einander gegenüberliegend angeordnete kreisförmige Durchgangsöffnungen 41 auf. In Längsrichtung 42 der Einführstange 5 ist im Bereich der beiden kreisförmigen Durchgangsöffnungen 41 ein Schlitz 43 vorgesehen. Der Schlitz 43 erstreckt sich bis zum distalen Ende der Buchse 4. Die Buchse 4 kann direkt am distalen Ende der Einführstange 5 ausgebildet oder separat aus einem anderen Material hergestellt und kraftschlüssig mit der Einführstange 5 verbunden, z.B. verklebt, werden.

Der Apnoe-Stent 1 weist an seinem proximalen Ende 2 eine Hülse 44 auf, an deren proximalem Ende ein kugelförmiges Verbindungselement 3 ausgebildet ist (Fig. 8, 10a, 10b). Das kugelförmige Verbindungselement 3 ist z.B. aus Edelstahl oder einem geeigneten Kunststoff ausgebildet. Der Durchmesser des kugelförmigen Verbindungselements 3 entspricht in der Größe nahezu dem äußeren Umfang der an der Einführstange 5 ausgebildeten Buchse 4. Die Hülse 44 wird bspw. durch Verkleben kraftschlüssig mit dem Apnoe-Stent 1 verbunden.

Das kugelförmige Verbindungselement 3 ist zwischen den beiden Durchgangsbohrungen 41 der Buchse 4 der Einführstange 5 aufnehmbar. Auf diese Weise wird eine lösbare Verbindung zwischen dem Apnoe-Stent 1 und der Einführstange 5 bereit gestellt. Der in der Buchse 4 ausgebildete Längsschlitz 43 erleichtert das Einführen und Herausziehen des kugelförmigen Verbindungselementes 3 aus der Buchse 4. Über das Verhältnis des Durchmessers des kugelförmigen Verbindungselements 3 zum Innendurchmesser der Buchse 4 sowie über die Härte des Materials für die Buchse 4 und die Länge des Schlitzes 43 ist die Verbindungs- bzw. Haltekraft des Verbindungselements 39 einstellbar. Das kugelförmige Verbindungselement 3 ist frei drehbar in der Buchse 4 gelagert. Durch eine derartige Ausbildung des Verbindungselementes 39 ergibt sich vorteilhaft, dass sich der Apnoe-Stent 1 im Einführschlauch 6 leicht und selbsttätig in die optimale Richtung entsprechend seiner aufgeprägten Form bzw. Biegung und der Form des Einführschlauchs ausrichtet. Die Form des Einführschlauchs ist an den Verlauf des Nasen- und Rachenraums angepasst. Hierdurch wird das Einführen erleichtert und Schmerzen für den Benutzer werden vermieden, da der Einführschlauch in seiner Form dem Verlauf des Nasen- und Rachenraums entspricht.

Die Buchse kann auch am Apnoe-Stent und das kugelförmige Verbindungselement dementsprechend an der Einführstange ausgebildet sein.

Über dem Apnoe-Stent 1 ist während des Einführens der Einführschlauch 6 angeordnet, um den Apnoe-Stent im Atemweg anzuordnen. Der Einführschlauch 6 weist an seinem distalen Ende vorzugsweise einen etwa kreissegmentförmig gebogenen Abschnitt auf, der sich etwa über einen Winkelbereich von ca. 80° bis 120° mit einem Radius von ca. 3 cm bis 7 cm auf (Fig. 1a, 9) erstreckt. Vorzugsweise ist der Einführschlauch aus einem ausreichend harten, geeigneten Kunststoff wie z.B. PEBAX mit einer Shore Härte von 60 Shore D bis 80 Shore D ausgebildet. Die Härte des Kunststoffs hat erheblichen Einfluss auf eine optimale bzw. exakte Anpassung des Einführschlauchs an den Nasengang während des Einführvorgangs. Am distalen Ende des Einführschlauchs 6 ist eine Einführspitze 45 einstückig angeformt, die sowohl im Innen- als auch im Außendurchmesser dem Einführschlauch 6 entspricht und mit diesem bündig ausgebildet ist. Die Einführspitze 45 kann aus einem flexiblen, wesentlich weicheren Material, wie z.B. PEBAX mit einer Shore Härte von 25 Shore D bis 45 Shore D ausgebildet sein. Der Einführschlauch 6 kann auch einteilig ausgebildet sein. Ein solcher einteiliger Einführschlauch kann aus einem einheitlichen Material, wie z.B. PEBAX72, bestehen oder es ist möglich, einen solchen einteiligen Einführschlauch mit einem Extrusionsverfahren mit kontinuierlichem Materialgradienten herzustellen. Bei einem solchen Einführschlauch gibt es einen Übergangsbereich, in dem kontinuierlich der Anteil des einen Materials abnimmt und dementsprechend der Anteil des anderen Materials zunimmt. Die Materialien eines solchen Schlauches sind z.B. PEBAX72 im Bereich des Hauptkörpers und PEBAX35 im Bereich der Einführspitze 45. Bzgl. der Materialauswahl gelten die obigen Ausführungen zum zweiteiligen Einführschlauch gleichermaßen.

Am distalen Ende der Einführspitze 45 des Einführschlauchs ist eine Fase 46 mit einem Winkel von in etwa 45° vorgesehen. Auf diese Weise werden Verletzungen der Gefäßwandungen beim Einführen des Apnoe-Stents 1 in den Atemweg vermieden und das Einführen wird erleichtert, da der Einführschlauch ideal den Biegungen des Atemwegs folgen kann und die Einführspitze sehr weich und flexibel ist. Der Außendurchmesser des Einführschlauchs beträgt bis zu 5 mm, um dem Benutzer eine komfortable, schmerzfreie Einführung zu ermöglichen. Der Innendurchmesser des Einführschlauchs beträgt bis zu 4 mm, um ausreichend Raum zur Aufnahme des Apnoe-Stents bereitzustellen. Durch die Krümmung des Einführschlauchs wird ein Anstoßen am Rachenraum verhindert und somit ein verletzungsfreies Einführen sichergestellt.

An Stelle der Fase kann die Einführspitze 45 auch mit einer Rundung ausgebildet sein. Eine solche Rundung kann bspw. mittels einer thermischen Umformung oder mittels Schleifen oder Laserabtragen erzeugt werden. Eine solche Rundung kann außenseitig und/oder innenseitig ausgebildet sein.

Eine solche abgerundete Spitze ist in der Herstellung aufwändiger als eine Fase. Die abgerundete Spitze ist jedoch noch sicherer beim Einführen des Stents. Zudem kann bei einer Fase das sich ergebende dünnwandige Ende verhärten, indem die Weichmacher aus dem Kunststoffmaterial durch die relativ große Oberfläche entweichen.

Diese geformten Einführspitzen 45 stellen atraumatische Spitzen dar, wobei je härter das Material der Spitze ist, desto glatter und runder sollte die Form sein.

Das Einführen des Apnoe-Stents kann darüber hinaus noch unterstützt werden, in dem der Apnoe-Stent 1 mit seinen runden Enden ein Stück (z.B. 2 - 4 mm) am Einführschlauch 6 beim Einführen vorsteht. Die runden Enden des Apnoe-Stents 1 bilden dann ein elastisches, atraumatisches Führungsmittel zum Einführen des Stents.

Nach dem Herausziehen des Stents 1 aus dem Atemweg ist dieser mit Körperflüssigkeiten, wie z.B. Nasenschleim, verunreinigt. Zum Reinigen des Apnoe-Stents 1 ist ein Reinigungsschlauch 47 vorgesehen. Der Reinigungsschlauch 47 ist vorzugsweise aus Kunststoff ausgebildet. Der Durchmesser des Reinigungsschlauches entspricht in etwa dem des Abschnitts des Apnoe-Stents 1 mit dem größten Durchmesser im aufgeweiteten Zustand. Vorzugsweise ist der Durchmesser des Reinigungsschlauches 47 etwas geringer als der des distalen Abschnitts des Apnoe-Stents 1, der den größten Durchmesser im aufgeweiteten Zustand aufweist. Die Oberfläche des Reinigungsschlauchs ist vorzugsweise satiniert. Durch eine derartige Beschichtung in Form einer Satinierung weist die Oberfläche des Reinigungsschlauchs eine geringere Oberflächenhaftung auf, so dass das Nitinolgeflecht durch Schieben leicht vom Reinigungsschlauch entfernt werden kann. Die Haftung ist aber dennoch hoch genug, um ein selbstständiges Herunterrutschen des Apnoe-Stents vom Reinigungsschlauch zu verhindern. Der Apnoe-Stent 1 kann einfach über dem Reinigungsschlauch 47 angeordnet und unter fließendem Wasser gereinigt werden. Vorteilhafterweise weist der Reinigungsschlauch 47 eine glatte Oberfläche auf, damit keinerlei Verunreinigungen daran anhaften können. Der Reinigungsschlauch kann auch als Stab aus einem Vollmaterial oder als Rohr ausgebildet sein. Als Material kann auch Glas vorgesehen sein.

Erfindungsgemäß ist es vorgesehen, den Apnoe-Stent 1 nach dem Reinigen mit einem Reinigungs- bzw. Dekontaminationsmittel zu reinigen, um Infektionen im Atemweg durch Verschleppung von am Apnoe-Stent 1 anhaftenden Keimen zu verhindern.

Ein Benutzer darf nicht mit Desinfektionschemikalien, insbesondere im Nasen- und Rachenraum, in Berührung kommen. Der Apnoe-Stent muss beim Reinigungsvorgang vollumfänglich, d.h. sowohl auf der Innen- als auch an der Außenfläche, mit dem Dekontaminationsmittel benetzt werden.

Deshalb muss das Reinigungsmittel zwar effektiv potentielle bakterielle, pilzliche und virale Verunreinigungen beseitigen, darf aber keine reizenden oder toxischen Effekte insbesondere im Nasen- und Rachenraum hervorrufen. Weiterhin muss es so ausgelegt sein, dass es möglichst selbsttätig und vollständig die feinen Drähte des Geflechts bzw. Gewebes bzw. Geleges des Apnoe-Stents 1 benetzt. Daher sind klassische Desinfektionsmittel wie z.B. Helipur®, Helix®, Stabimed®, etc. von B.Braun Melsungen oder Gigasept Instru AF® von Schülke & Mayr GmbH, die zumeist aldehyd- oder phenolbasiert sind, nicht geeignet. Enzymatische Reiniger wie z.B. Helizyme®, etc. von B.Braun Melsungen sind nicht dekontaminierend wirksam und daher gleichfalls nicht geeignet, wenn auch hautverträglicher. Die gängigen Wundantiseptikmittel PVP-Iod und Octenidin sind wegen ihrer schleimhautreizenden und zelltoxischen Wirkung gleichfalls nicht geeignet.

Ein in der Wundbehandlung und Infektionsprophylaxe (insbesondere auch gegen multiresistente Staphylococcus aureus-Bakterien, "MRSA") beim Menschen bekanntes wirksames und gut verträgliches Mittel ist Polyhexanidlösung in optimierter Zusammensetzung z.B. Prontoderm®, Prontosan® von B.Braun Melsungen oder ProntoMan®, ProntoLind® von Prontomed GmbH erhältlich.

Polyhexanid (-Polyaminopropyl-Biguanid) ist ein kationisches Biguanid mit breiter mikrobiozider Wirksamkeit. Es bietet eine effektive Keimreduzierung und ein breites Wirkspektrum, auch bei längerem Einsatz. Die mikrobiozide Wirkung beruht in erster Linie auf einer selektiven Beeinflussung der Durchlässigkeit der bakteriellen Zellmembran durch starke Wechselwirkung gegenüber sauren Lipiden bakterieller Zellmembranen. Hieraus resultiert eine gute Gewebeverträglich beim Menschen.

Die erfindungsgemäß vorzugsweise einzusetzende Lösung beinhaltet neben dem Wirkstoff Polyhexanid in abgestimmter Menge ein Betain als Detergens. Eine solche Polyhexanidlösung, die zusätzlich Undecalenamidopropyl-Betain enthält, hat sich als gut geeignet erwiesen für die Dekontamination des Apnoe-Stents. Die Reduzierung einer bakteriellen Testanschmutzung beträgt bis zu sieben Log-Stufen und liegt damit über der bekannten von fünf Log-Stufen für die Anwendung am Menschen. Durch das in der Lösung enthaltene Betain kann die Polyhexanidlösung sehr effizient als Spray für die Behandlung des Apnoe-Stent 1 eingesetzt werden. Das Betain reduziert die Oberflächenspannung so stark, dass sich die minimalen Mengen aufgesprühter Polyhexanidlösung vollflächig um die Drähte herum anlegt, so dass auch insbesondere auf der nach innen gewandten, nicht direkt angesprühten Seite des Apnoe-Stents 1 eine wirksame Dekontamination erfolgt. Eine eingetrocknete Testanschmutzung mit der hohen Anzahl von 10⁹ Bakterien wurde bei Reinigungstests durch die Polyhexanidlösung um mindestens 5 Log-Stufen reduziert. Bevorzugt wird Polyhexanid in einer Konzentration von 0,01% bis bis 0,3%, noch mehr bevorzugt 0,1% bis 0,3%, am meisten bevorzugt 0,25% eingesetzt, um auch eine desinfizierende Wirkung zu erreichen. Das Undecalenamidopropyl-Betain wird vorzugsweise in einer Konzentration von 0,05% bis 0,5%, noch mehr bevorzugt 0,10% bis 0,15% eingesetzt.

Durch das Reinigungsverfahren in drei Schritten mit Abspülen der Verunreinigungen unter warmem Wasser, mechanischer Reinigung auf dem Reinigungsschlauch und nachfolgendem Einsprühen mit Polyhexanid-Betain-Lösung wird ein desinfizierender Effekt erreicht, der die Verwendung des Apnoe-Stent bei verschiedenen Patienten ermöglicht.

Nachfolgend sind alternative Ausführungsformen der oben beschriebenen Bauteile angegeben.

Die Klemmbacken 11, 12 der Fixiereinrichtung der erfindungsgemäßen Fixiervorrichtung 10 können auch nach dem Wirkprinzip einer Curry-Klemme ausgebildet sein. Eine Curry-Klemme umfasst zwei drehbare, auf parallelen Achsen angebrachten Kunststoff- oder Metallbacken, zwischen denen das proximale Ende des Apnoe-Stents angeordnet werden kann. Die Backen sind vorzugsweise exzentrisch geformt und werden durch Federn gegeneinander gepresst. Zur Verstärkung der Klemmwirkung weisen ihre Auflageflächen ein strukturiertes Profil auf.

Wird der Apnoe-Stent in der Curry-Klemme angeordnet, ist er gegen ein Hineinrutschen in den Atemweg gesichert, da die Backen den Stent einklemmen. Zum Lösen kann der Stent nach oben aus der Klemme herausgezogen werden. Allerdings wird der Stent dann nur gegen ein zu tiefes Eindringen in den Atemweg gesichert.

Das Verbindungselement 39 kann beispielsweise auch als Bajonettverschluss ausgebildet sein. Der Bajonettverschluss umfasst zwei Verbindungselemente, wobei ein erstes Verbindungselement über einem zweiten Verbindungselement anordbar ist. Das erste Verbindungselement, das über das zweite Verbindungselement geschoben wird, besitzt einen Längsschlitz, an dessen Ende sich rechtwinklig ein kurzer Querschlitz ansetzt. Das zweite Verbindungselement weist eine Nase auf, die in den Querschlitz eingeführt wird und dann die feste Verbindung bewirkt. Die Verbindung erfolgt über eine Steck-Dreh-Bewegung. Beim Verbinden werde die beiden Verbindungselemente ineinandergesetzt. In etwa senkrecht zur Steckrichtung sind in beiden Verbindungselementen an der Verbindungsstelle längliche Erhebungen angebracht.

Diese laufen jedoch nicht rundum, sondern sind unterbrochen. Da die Erhebungen nun leicht schräg in der Ebene senkrecht zur Steckrichtung liegen, werden durch eine Drehbewegung beide Verbindungselemente gegeneinander gepresst. Es sind auch andere Schiebemechanismen möglich.

Auch eine Verbindung über ein Gewinde, einen Clipverschluss oder eine Kombination daraus ist möglich.

Die Aufnahme kann auch als Clipverschluss oder Druckknopf oder dergleichen ausgebildet sein.

**Bezugszeichenliste**

| | | | |
|---|---|---|---|
| 1 | Apnoe-Stent | 25 | Fixierblock |
| 2 | proximales Ende | 26 | langer Schenkel |
| 3 | Kopplungselement, Kugel | 27 | Oberseite |
| 4 | Kopplungselement, Buchse | 28 | Unterseite |
| 5 | Einführstange | 29 | Seitenwandung |
| 6 | Einführschlauch | 30 | Gelenkseite |
| 7 | distales Ende | 31 | Gelenkkörper |
| 10 | Fixiervorrichtung | 32 | Durchgangsöffnung |
| 11 | erste Klemmbacke | 33 | Fixierschenkel |
| 12 | zweite Klemmbacke | 34 | Durchgangsloch |
| 13 | Gelenk | 35 | Fixierschlauch |
| 14 | Basiswandung | 36 | Rastnase |
| 15 | Oberseite | 37 | Befestigungseinrichtung |
| 16 | Unterseite | 39 | Verbindungselement |
| 17 | Seitenwandungen | 41 | Durchgangsöffnung |
| 18 | Gelenkseite | 42 | Längsrichtung |
| 19 | Verschlussseite | 43 | Schlitz |
| 20 | Ringscheibenelemente | 44 | Hülse |
| 21 | Durchgangsöffnung | 45 | Einführspitze |
| 22 | Rohrwelle | 46 | Fase |
| 23 | Verschlusskante | 47 | Reinigungsschlauch |
| 24 | U-förmige Ausnehmung | | |

## Patentansprüche

1. Einführschlauch zum Einführen eines Apnoe-Stents, wobei der Einführschlauch (6) am distalen Ende (7) ein Kreissegment aufweist, um das Einführen in den Atemweg zu erleichtern,
**dadurch gekennzeichnet,**
**dass** das Kreissegment eine Biegung von etwa 80°- 120°, insbesondere von 90° bis 100° und vorzugsweise von 95° aufweist, wobei ein Radius von etwa 3 cm bis 7 cm und vorzugsweise vom 5 cm vorgesehen ist.

2. Einführschlauch zum Einführen eines Apnoe-Stents nach Anspruch 1,
**dadurch gekennzeichnet,**
**dass** am distalen Ende des Einführschlauchs (6) eine Einführspitze (45) einstückig angeformt ist.

3. Einführschlauch zum Einführen eines Apnoe-Stents nach Anspruch 2,
**dadurch gekennzeichnet,**
**dass** die Einführspitze (45) aus einem flexiblen weicheren Material als der Hauptkörper des Einführschlauchs (6) ausgebildet ist.

4. Einführschlauch zum Einführen eines Apnoe-Stents nach Anspruch 2 oder 3,
**dadurch gekennzeichnet,**
**dass** die Einführspitze (45) am distalen Ende ein Fase (46) oder eine Rundung aufweist.

5. Einführschlauch zum Einführen eines Apnoe-Stents nach Anspruch 4,
**dadurch gekennzeichnet,**
**dass** die Fase (46) mit einem Winkel von etwa 45° vorgesehen ist.

6. Einführschlauch zum Einführen eines Apnoe-Stents nach Anspruch 3,
**dadurch gekennzeichnet,**
**dass** der Hauptkörper des Einführschlauchs (6) aus einem Plastikmaterial mit einer Shore-Härte von 60 Shore D bis 80 Shore D ausgebildet ist.

7. Einführschlauchs zum Einführen eines Apnoe-Stents nach Anspruch 3,
**dadurch gekennzeichnet,**
**dass** die Einführspitze (45) aus einem Plastikmaterial mit einer Shore-Härte von 25 Shore D bis 45 Shore D ausgebildet ist.
